# EUROPEAN PATENT SPECIFICATION

(11) **EP 3 927 268 B1**
(45) Date of publication and mention of the grant of the patent: **01.11.2023**
(21) Application number: 20718518.2
(22) Date of filing: 27.03.2020
(51) Int. Cl.: A61B 18/18, H05B 6/70

(54) **MICROWAVE APPARATUS AND METHOD**
MIKROWELLENVORRICHTUNG UND VERFAHREN
APPAREIL ET PROCÉDÉ À MICRO-ONDES

(30) Priority: 29.03.2019 GB 201904383
(43) Date of publication of application: 29.12.2021
(73) Proprietor: Emblation Limited, Inglewood, Alloa FK10 2HU (GB)
(72) Inventor: MCERLEAN, Eamon, Inglewood, Alloa FK10 2HU (GB); BEALE, Gary, Inglewood, Alloa FK10 2HU (GB); JOSHI, Shailesh, Inglewood, Alloa FK10 2HU (GB)
(74) Representative: Marks & Clerk LLP
(86) International application number: PCT/EP2020/058840
(87) International publication number: WO 2020/201149

(56) References cited:
- EP-A1- 2 221 921
- WO-A1-2016/014320
- WO-A1-2017/008020
- DE-A1-102017 121 732

## Description

### Field

The present invention relates to a microwave antenna apparatus and method, for example a microwave antenna apparatus in which a reactive element provides both impedance matching and reduction or removal of surface currents.

### Background

It is known to use an interstitial antenna in a medical energy system to deliver energy into biological tissues for ablative or non-ablative purposes. In most energy ablation systems the energy is delivered from an energy generator, via a connecting cable, to a radiating applicator that transfers the energy into the tissue. In these applicators, the radiating element is surrounded by tissue or is placed in contact with the tissue. For such systems, a standard practice is to deliver energy for a treatment lasting typically anywhere from 1 to 20 minutes to raise the temperature of tissue greater than 43 to 45°C, for example to 60, 70 or 100°C and beyond. The temperature of the tissue may be raised such that necrosis occurs within a desired ablation zone. The energy may be delivered to have an amplitude-modulated or pulse width-modulated duty cycle to ensure that a required level of energy is maintained or controlled for the duration of the energy release.

One undesired aspect of treatment may be the presence of radiation from antenna surface currents. Radiation from antenna surface currents may result in the heating or ablation of adjacent healthy tissue beyond the desired target ablation zone. In some circumstances, the additional radiation can skew an ideal isotropic (for example, spherical) zone of necrosis into a more lachrymiform (tear drop) shape which may be an issue when planning ablations.

Typically, ablation antennas are formed around a monopole or dipole methodology utilising an arrangement of the inner and outer conductors of a coaxial transmission line to produce radiating elements that match to the impedance of the tissue target. Alternating currents flowing in the antenna result in an electromagnetic wave being generated and radiated into the surrounding tissue. The currents flowing in the antenna element connected or coupled to the transmission line outer conductor return to the generator by flowing on the outer conductor of the coaxial transmission line.

Currents that return to the generator by flowing down the outer conductor of the coaxial transmission line may be referred to as common mode currents. These common mode currents may induce radiation from the outer conductor of the coaxial transmission line which may distort the antenna radiation pattern. The overall electrical length of the transmission line may have significant influence on the susceptibility of the transmission line to common mode currents.

A balun may be used to balance even and odd mode currents between the radiating element and the transmission line outer conductor. A balun works by matching the phase of a balanced radiating element to an unbalanced coaxial feed line to prevent common mode currents flowing. Preventing common mode currents flowing may ensure a uniform field pattern.

One of the limitations of a balun may be that the balun may comprise quarter-wavelength sections, which may place a spatial constraint upon the design. It is known that dielectric loading can be used to reduce the physical size of a transmission line for the same electrical length. Dielectric loading is often employed to maintain compact geometries. Dielectrics that are commonly used include various microwave ceramics, for example alumina, zirconia or water jackets. Typically, dipole antennas are balanced antennas whereas monopole antennas are unbalanced. In the case of monopole antennas, an unun as opposed to a balun may be used to match an unbalanced line to an unbalanced radiating element.

Another method to reduce surface currents is to form a conductive sleeve or shield around the outer ground-plane. The conductive sleeve or shield traps and reflects the surface currents, utilising phase to cancel the unwanted surface currents. A conductive sleeve or shield may be referred to as a choke or as a sleeve balun or bazooka balun. A choke or sleeve or bazooka balun may typically be grounded to the outer conductor to create quarter-wavelength sections and may be filled with a dielectric to further reduce the size.

Methods described above may have geometric requirements that may significantly increase the complexity and fabrication of the antenna structure, for example by physically increasing the diameter of the antenna structure to accommodate all the components required to create the balun or choke mechanisms. EP 2221921 describes a device for directing energy to a target volume of tissue. WO 2017/008020 describes an electrode designed as an RF/microwave antenna. WO 2016/014320 describes a system for extraction of volatiles from bodies in a vacuum.

### Summary of the invention

In a first aspect, there is provided a microwave apparatus as defined by claim 1.

The microwave feed line may comprise a coaxial cable. The ground may comprise an outer conductor of the coaxial cable.

The reactive element may comprise at least one aperture formed in the outer conductor of the coaxial cable. The aperture may be formed by selective removal of part of the outer conductor.

The at least one aperture may comprise at least one longitudinal slot.

At least one conductive strip may remain between the longitudinal slot or slots. The at least one conductive strip may form an inductive conductor element.

The reactive element may further comprise at least one conductive wire positioned across the at least one longitudinal slot, thereby forming an inductive conductor element.

The reactive element may comprise at least one capacitive ring at a distal end of the coaxial cable. The or each capacitive ring may comprise a ring of outer conductor material remaining after formation of the at least one aperture.

The at least one capacitive ring may be electrically connected to the at least one inductive conductor element.

The inductive conductor element may comprise at least one discontinuity along the length of the inductive conductor element. The at least one discontinuity may provide a capacitance.

The at least one longitudinal slot may vary in width along the length of the slot.

The at least one longitudinal slot may have a stepped width such that different portions of the longitudinal slot have different widths.

The at least one longitudinal slot may be a single longitudinal slot.

The least one longitudinal slot may be a radially opposed pair of longitudinal slots.

The radiating element may comprise a monopole antenna.

The radiating element may comprise an exposed distal portion of an inner conductor of the coaxial cable, the inner conductor of the coaxial cable being longer than an outer conductor of the coaxial cable.

The surface currents may comprise common mode currents.

The microwave apparatus may be configured to perform microwave ablation of tissue at the operational frequency or range of frequencies. The microwave apparatus may be configured to provide tissue hyperthermia at the operational frequency or range of frequencies.

Claim 11 defines a microwave system comprising: a microwave generator; a controller configured to control the microwave generator to generate microwave energy having a selected operational frequency or range of frequencies; a microwave feed line configured to deliver the microwave energy to a radiating element extending from or coupled to a distal end of the microwave feed line; the radiating element; and a reactive element formed in or on the microwave feed line; wherein the operational frequency or range of frequencies is selected such that the reactive element both provides a desired degree of match between an impedance of the radiating element and an impedance of the microwave feed line and reduces or eliminates surface currents flowing on a ground of the feed line.

Claim 12 defines a method comprising controlling a microwave generator to generate microwave energy having a selected operational frequency or range of frequencies; and delivering by a microwave feed line the microwave energy to a radiating element extending from or coupled to a distal end of the microwave feed line, wherein a reactive element is formed in or on the microwave feed line; wherein the operational frequency or range of frequencies is selected such that the reactive element both provides a desired degree of match between an impedance of the radiating element and an impedance of the microwave feed line and reduces or eliminates surface currents flowing on a ground of the feed line.

Claim 13 defines a method of designing a microwave apparatus, the method comprising: simulating operation at a selected frequency or range of frequencies of a radiating element extending from or coupled to a distal end of a microwave feed line; and performing an iterative design procedure comprising: simulating detuning of the radiating element; and selecting reactive properties of a reactive element formed in or on the microwave feed line, the iterative design procedure being repeated until at the selected frequency or range of frequencies the reactive properties of the reactive element provide a desired degree of match between a simulated impedance of the radiating element and a simulated impedance of the microwave feed line while reducing or eliminating simulated surface currents flowing on a ground of the feed line.

In a further aspect, which is not a part of the present invention, there is provided a method of fabricating a microwave apparatus, the method comprising: providing a coaxial cable; at a distal end of the coaxial cable, selectively removing a distal portion of the outer conductor of the coaxial cable to expose a distal portion of the inner conductor to form a radiating antenna element; selectively removing at least one further portion of the outer conductor of the coaxial cable, thereby forming at least one aperture in the outer conductor of the coaxial cable, wherein parameters of the at least one aperture are selected to provide a desired degree of match between an impedance of the radiating element and an impedance of the microwave feed line and to reduce or eliminate surface currents flowing on a ground of the feed line, when operated at a selected operational frequency or range of frequencies.

The at least one aperture may comprise at least one longitudinal slot.

The selective removing of the at least one further portion of the outer conductor may comprise at least one of: sawing, slicing, cutting, burning, melting, eroding, planing, polishing, hydroforming, machining, laser cutting, etching, acid erosion.

An energy control mechanism is described to prevent unbalanced currents heating an antenna surface.

The method includes an electromagnetic energy generator system, cabling and applicator used to deliver energy from the generator system to a recipient device, for example a radiating applicator antenna that transfers the energy into biological tissue.

Energy is delivered to a radiating element utilising a high impedance discontinuity to effect both an impedance transformation to match the radiating element to the transmission channel whilst simultaneously utilising the same region to limit the return of surface currents onto the outer conductor of the transmission line.

A more compact antenna for the delivery of microwave energy into tissue may be provided. A simple technique may be used to match the antenna to the feed line, which may reduce the common mode currents and may not be overly complex to manufacture.

In order to match the antenna to the feed line an impedance transformer may be used. Matching networks may be constructed around tuning elements such as stubs, quarter-wavelength transformers (phase and impedance) or reactive tuning features. Inductive reactance may usually be formed by centre conductor length. Capacitive reactance may commonly be controlled by coaxial spacing. Combinations of inductive reactance and capacitive reactance may be used to change impedance. Typical reactive matching may include T and Pi network structures. Balanced Pi arrangements (for example, O-Pad structures) with inductive ground elements may have enhanced benefits as they place inductive reactance in both the signal line and the ground line that may act like a common-mode choke.

This means for example that standard antenna designs may match an ideal antenna to a transmission line feed by one method and then may employ a secondary method to deal with common mode currents.

An approach described herein, which may be more efficient, comprises creating a single structure, element, feature or method that supports the unbalanced impedance matching by utilising return energy from an antenna mismatch to cancel out a common mode current. This means that the reactive properties of the feed line and antenna mismatch may be simultaneously adjusted to purposely and destructively cancel.

Inductive signal and ground elements are included in tandem with capacitive ground elements to create a combined matching-choke feature.

In one aspect, a microwave therapy needle comprises: a microwave antenna having a radiating element and adapted to deliver microwave energy to a target tissue; and a reactive property in the feed line ground; and utilises mismatch of a radiating element in conjunction with the reactive property to simultaneously match and choke surface currents normally present on the feed line.

The reactive property may be realised by an inductive conductor electrically connected across a capacitive element formed in the feed line outer conductor.

The reactive capacitive property may be realised by a slot of conductive material removed from the outer conductor.

The reactive capacitive property may be further realised by a conductive ring or collar on the antenna outer conductor.

The reactive inductive property may be realised by a thin conductor formed in the outer conductor.

The reactive inductive property may be realised by a thin wire attached electrically to the outer conductor.

The reactive inductive property may electrically connect to the capacitive ring.

The capacitive ring may be adjacent to, close to, or at the feed point of the radiating element.

The radiating element may be separate from the reactive property but is designed to work in conjunction with it.

The reactive property may present a high impedance to surface currents on the coaxial feed by cancellation against the antenna mismatch.

There may also be provided an apparatus or method substantially as described herein with reference to the accompanying drawings.

Any feature in one aspect of the invention may be applied to other aspects of the invention, in any appropriate combination. For example, apparatus features may be applied to method features and vice versa.

### Brief Description of Drawings

Embodiments of the invention are now described, by way of non-limiting examples, and are illustrated in the following figures, in which:-
Figure 1 is a diagrammatic equivalent circuit illustrating characteristics of a matching-choke in accordance with an embodiment;
Figure 2 is an illustration of a monopole antenna that includes a dual conductor matching-choke in accordance with an embodiment;
Figure 3 is a drawing indicating a number of conductor configurations in accordance with embodiments;
Figure 4 is a drawing of a cross-sectional longitudinal cut of a monopole antenna variant that includes a dual conductor matching-choke in accordance with an embodiment;
Figure 5 is an illustration of a monopole antenna variant that includes a single conductor matching-choke in accordance with an embodiment;
Figure 6 is a drawing of a cross-sectional longitudinal cut of a monopole antenna variant that includes a single conductor matching-choke in accordance with an embodiment;
Figure 7 is a drawing of a cross-sectional longitudinal cut of a monopole antenna variant that includes a tri-conductor matching-choke in accordance with an embodiment;
Figure 8 is an illustration of monopole antenna variants that include shaped/stepped matching-choke element in accordance with embodiments;
Figure 9 is an illustration of a monopole antenna variant that includes an inductive/capacitive discontinuity in accordance with an embodiment;
Figure 10 is an illustration of a monopole antenna variant that includes multiple inductive/capacitive discontinuities in accordance with an embodiment;
Figure 11 is an illustration of a monopole antenna variant that includes multiple stepped inductive/capacitive discontinuities in accordance with embodiments;
Figure 12 is an illustration of monopole antenna variants with a matching-choke in accordance with embodiments;
Figure 13 is a simulated plot of the SAR pattern at 2.45 GHz for a monopole antenna without a matching-choke element;
Figure 14 is a simulated plot of the S11 for a monopole antenna without a matching-choke element;
Figure 15 is a simulated plot of the necrosis pattern at 2.45 GHz for a monopole antenna without a matching-choke element;
Figure 16 is a simulated plot of the SAR pattern at 2.45 GHz for a monopole antenna with a matching-choke element;
Figure 17 is a simulated plot of the S11 for a monopole antenna with a matching-choke element
Figure 18 is a simulated plot of the necrosis pattern at 2.45 GHz for a monopole antenna with a matching-choke element;
Figure 19 is a comparison of simulated plots of the necrosis pattern at 2.45 GHz for a monopole antenna with and without a matching-choke element;
Figure 20 is a photograph of a monopole antenna with a matching-choke element;
Figure 21 is a macro-photograph of the inductive conductor from a matching-choke element;
Figure 22 is a set of photographs of monopole antenna with a matching-choke element constructed using wire conductors;
Figure 23 is a set of photographs of antenna ablation patterns created by a monopole with a matching-choke element;
Figure 24 is a set of photographs of antenna ablation patterns created by a monopole with a matching-choke element; and
Figure 25 is a schematic illustration of a system in accordance with an embodiment.

### Description of the invention

An antenna component that both provides impedance matching and reduces or removes surface currents may be referred to as a matching-choke. The matching-choke may provide a high impedance discontinuity to effect an impedance transformation to match the radiating element to the transmission channel. The matching-choke may simultaneously limit the return of surface currents onto the outer conductor of the transmission line.

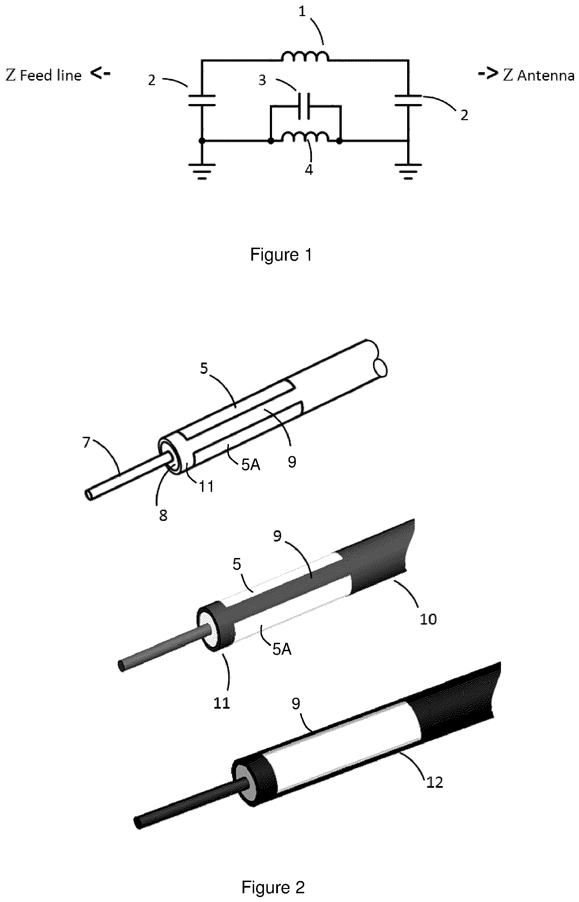

An equivalent circuit representing an antenna matching-choke is illustrated in the circuit diagram of Figure 1.

In the embodiment of Figure 1, an antenna comprises a coaxial feed, a radiating element and a matching-choke. The antenna may be, for example, an antenna as described below with reference to Figure 2. In the embodiment of Figure 2, a monopole radiating element 7 is formed from a coaxial cable by cutting back a part of the outer conductor and dielectric 8 of the coaxial cable such that an exposed section of the inner conductor protrudes to act as the monopole radiating element 7. The section of the coaxial cable extending to the base of the monopole radiating element 7 acts as a coaxial feed which provides energy to the monopole radiating element 7.

In this simplified circuit, a series inductance of the coaxial feed is represented by the inductor 1. A series capacitance of the coaxial feed is represented by capacitance 2. The circuit also includes a reactive element in parallel with the coaxial feed line inductance. The reactive element is representative of a reactive property of the matching-choke. The reactive element comprises a capacitive property 3 and an inductive property 4.

In the embodiment shown in Figure 2, the reactive LC element is realised as a shorted-slot in the coaxial outer conductor. The shorted-slot feature creates a high impedance point in the circuit that both suppresses surface current and matches the antenna impedance (Z Antenna) with the coaxial feed line impedance (Z Feed line). For example, the coaxial feed line may be 50 Ohm or 75 Ohm or any other standard coaxial feed impedance. The antenna impedance may be any impedance, for example any impedance from 70 Ohm to 300 Ohm. The antenna impedance depends upon the design of the antenna and the tissue into which it is intended to radiate. The matching of the impedances may be such as to provide a desired degree of match, for example to achieve an antenna return loss of between 12 dB and 15 dB.

An example of a realisation of the matching-choke is illustrated in Figure 2. Figure 2 comprises three views of the same antenna. In the first (top) view, components of the antenna are represented in outline. In the second (middle) and third (bottom) views, conductive components are shown in black to distinguish over non-conductive components. The third view shows the antenna rotated approximately 90 degrees around its longitudinal axis as compared to the first and second views.

In the embodiment of Figure 2, the matching-choke is applied to a simple monopole antenna. In other embodiments, the matching-choke may be applied to any other antenna type, not limited to a monopole.

The radiating monopole 7 is separated from the outer conductor of the coaxial feed by an insulating dielectric 8 of the coaxial feed. The radiating monopole 7 is detuned to present a mismatch between the radiating monopole 7 and the coaxial feed that will be used by the matching-choke.

The metal conductive parts are illustrated by 9 (inductive feed), 11 (capacitive ring) and 10 (coaxial outer conductor). The slot 5 in Figure 2 is an area in which the outer conductor of the coaxial feed has been removed to expose the inner dielectric 8, changing its inductive characteristic. In the present embodiment, at least some of the inner dielectric 8 remains in place when the area of the outer conductor is removed. In further embodiments, an area of the inner dielectric 8 is also removed to expose part of the inner conductor. Exposing of the inner conductor may be of benefit if a high dielectric material is located in proximity to the exposed portion. In some circumstances, proximity to a high dielectric material may be exploited to reduce the size of the slot.

The slot 5 extends longitudinally along a portion of the coaxial feed. The slot 5 extends around the circumference of the coaxial feed by, for example, around 120 to 150 degrees. A further slot 5A is positioned on the opposite side of the coaxial feed from the slot 5 and has the same proportions as the slot 5.

Slots 5 and 5A do not extend to the distal end of the coaxial feed. When part of the outer conductor is removed to form slots 5 and 5A, a ring of outer conductor material remains at the distal end of the coaxial feed. The ring may be referred to as a capacitive ring 11.

The parallel inductive component shown in the circuit diagram as inductive component 4 is generated by a conductive element 9 which may be referred to as an inductive feed. The conductive element 9 joins the main body of the outer conductor of the coaxial cable to the ring element 11 near the monopole feed point. The conductive element 9 is formed of the part of the outer conductor that remains between the slots 5 and 5A. A further conductive element 12 is present on the opposite side of the coaxial feed.

The slots 5 and 5A formed in the outer conductor and corresponding ring element 11 create the capacitive properties that balance with the inductive elements 9 and 12 to match the monopole to the coaxial feed line. Simultaneously, the inductive elements 9 and 12 provides a high impedance path to suppress the surface currents that would flow on the outer conductor 10. In this embodiment there are two conductive strips 9 and 12 placed diametrically to provide a balanced pair of inductive conductors. The inductive conductors 9, 12 may not interfere with the roundness of the radiated field in the tangential axes since the inductive conductors 9, 12 are disposed away from the radiating element 7.

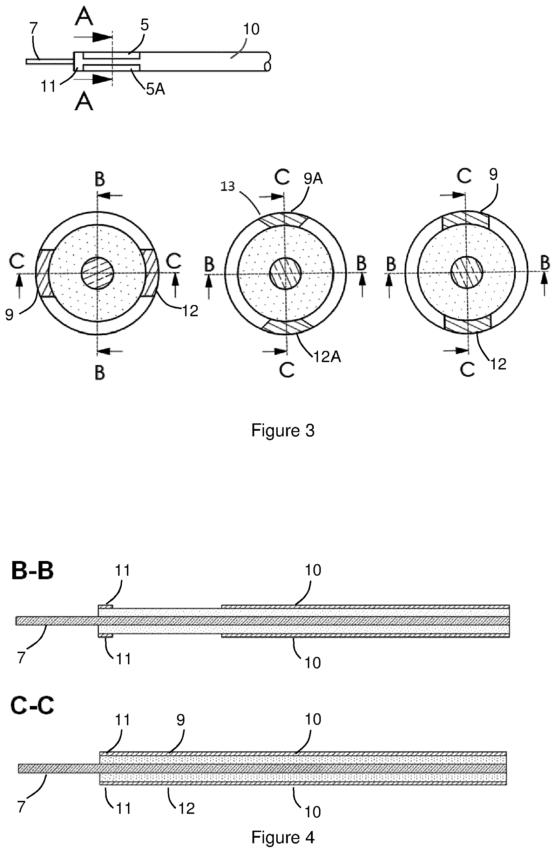

A number of cross sections of the antenna of Figure 2 are presented in Figure 3. The first (top) view of Figure 3 shows a side view of the antenna. The second (bottom left) and fourth (bottom right) views each show an axial cross section of the antenna of Figure 2. The third (bottom middle) view shows an axial cross section of a similar antenna in which material of the outer conductor is removed by a different method, forming a different profile of the remaining conductive strips.

In the embodiment of Figure 2, the conductors 9 and 12 are created by removing a portion of the outer conductor material to leave a reduced width conductive element 9, 12 remaining. This material may be removed to be perpendicular to the middle axis for example by sawing, slicing, cutting, burning, melting, eroding, planing, polishing, hydroforming, machining, laser cutting, or any other method to remove it.

In the example shown in the third view of Figure 3, material may also be removed to be at an arbitrary angle 13 at the edges of conductors 9A, 12A. The angle 13 may represent an undercut from an etching or acid erosion process. In further embodiments, the coaxial cable may be fabricated with the gaps 5, 5A already in place.

Figure 4 shows two further cross sections of the antenna of Figure 2. Figure 4 indicates for clarity that the conductive coaxial structure in that example exists only in one axis and is discontinued in the other orthogonal axis. The first (upper) cross section shows a cross section through the slots 5, 5A, demonstrating the presence of the ring 11 and remaining outer conductor 10. The second (lower) cross section is taken through the conductive strips 9, 12 and so the outer conductor appears continuous along the length of the coaxial feed in this cross section.

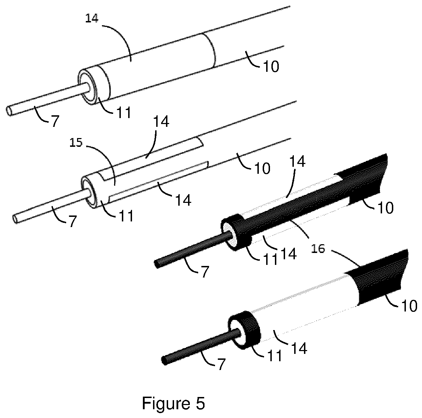

A further embodiment having a single conductive strip 17 is illustrated in Figure 5. Figure 5 comprises four views. From top to bottom these comprise a first orientation of the antenna shown in outline; a second, rotated orientation of the antenna shown in outline; a rotated orientation in which conductive material is shown in black; and the first orientation in which conductive material is shown in black.

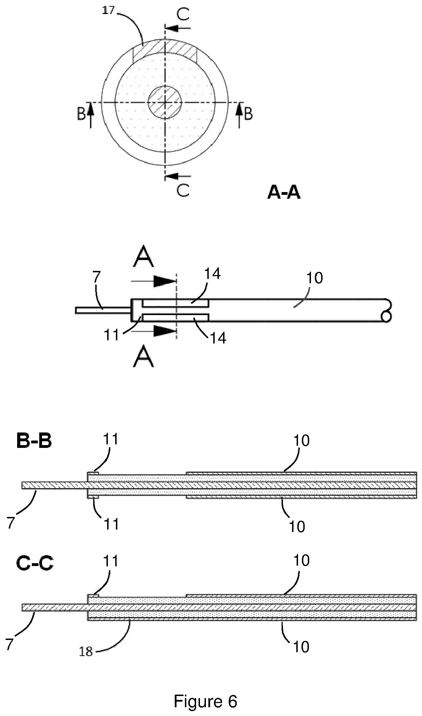

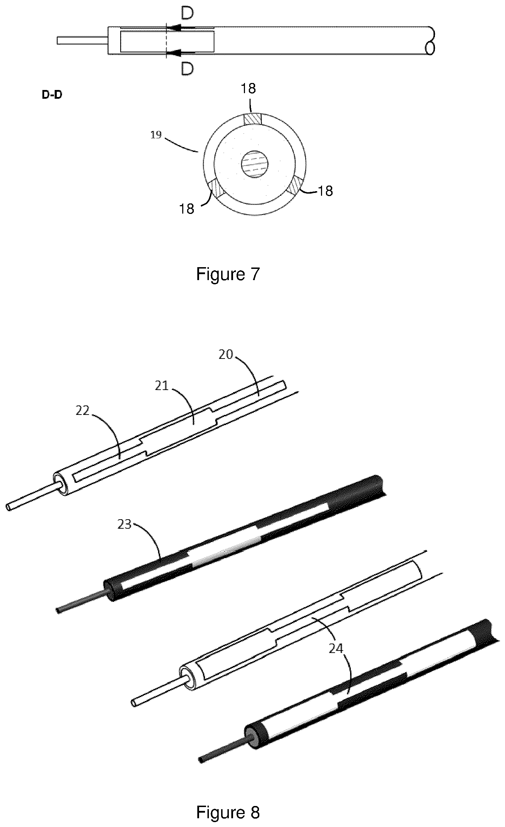

In the embodiment of Figure 5, a single slot 14 is formed in the outer conductor. The single slot 14 may extend around the circumference of the coaxial feed by, for example, 280 to 310 degrees. A slot length of 302.7 degrees will provide a conductor width equal to the cable radius. This configuration may also be used to simplify the construction. In some circumstances, the roundness of the radiation pattern may be minimally impacted by imbalance by the lack of symmetry in the construction of the antenna of Figure 5, as indicated in Figure 6. Figure 6 comprises four views which are now described from top to bottom. The first (top) view of Figure 6 is an axial cross section of the antenna of Figure 5, showing the single conductive strip 17. A second view of Figure 6 is a side view of the antenna of Figure 5. The third view and fourth (bottom) view of Figure 7 are longitudinal cross sections taken at perpendicular angles.

Another variant is presented in Figure 7 with a tri-conductor arrangement that may provide enhanced tri-axial symmetry or balance the mechanical forces between the outer conductor and the capacitive ring 11. Three conductive strips 18 are positioned around the circumference of the coaxial cable. The three conductive strips 18 are positioned between three slots 19. The first (top) view of Figure 7 shows a side view of the antenna of Figure 7. The second (bottom) view of Figure 7 shows an axial cross-section.

The arrangement of inductive conductors may also include stepped sections to add additional capacitance as illustrated in Figure 8. Figure 8 comprises four views which are now described from top to bottom. The first (top) view and second view show a first stepped-slot embodiment in outline (first view) and with conductive material represented in black (second view). In the first stepped-slot embodiment, a slot comprises a wide section 21 in the centre of the slot and narrow sections 22, 20 at the ends of the slot. The remaining conductive material is shown as 23.

The third view and fourth (bottom) view of Figure 8 show a second stepped-slot embodiment in outline (third view) and with conductive material shown in black (fourth view). The slot of Figure 8 is narrow in the centre 24 and wider at the sides 24A, 24B. In other embodiments, the slot may widen or narrow at one side only. Any number or order of steps may be used. In some embodiments, the stepped slot may add more capacitance to the arrangement and/or may enhance the bandwidth of the matching between antenna and feed line.

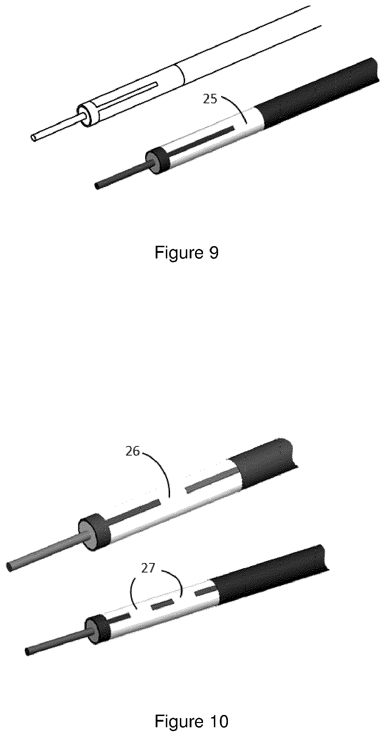

Other embodiments include an inductive element with a capacitive discontinuity 25 as shown in Figure 9 in outline (top view) and with conductive material shown in black (bottom view) The capacitive discontinuity 25 is formed by making a break in a conductive strip between slots. In the embodiment of Figure 9, the break 25 is at the proximal end of the conductive strip. In this case the break in the inductive line may further enhance the capacitive properties of the arrangement.

A capacitive discontinuity may also exist at a number of other regions. Figure 10 shows two further embodiments having capacitive discontinuities. In the first embodiment of Figure 9 (shown in the top view), the capacitive discontinuity 26 is in the centre of the strip. Multiple discontinuities 27 may be created to filter or tune the bandwidth of performance. The second embodiment shown in Figure 10 (bottom view) shows two capacitive discontinuities 27 in a single capacitive strip. In other embodiments, any suitable number or positioning of capacitive discontinuities may be used.

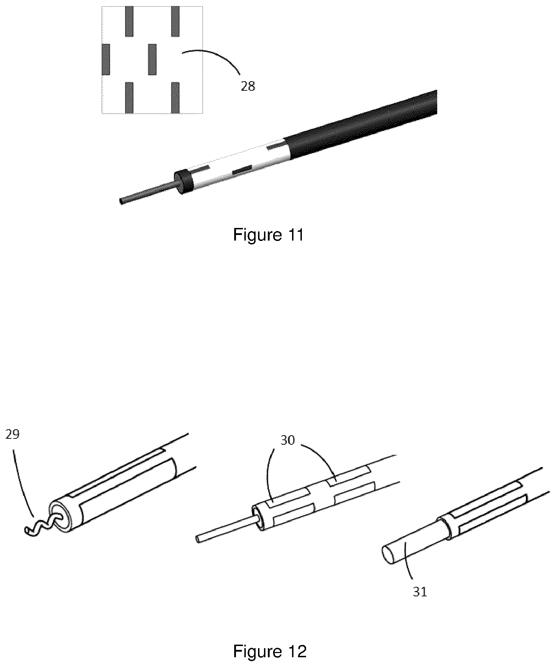

The discontinuities may be further spaced in any stepped arrangement 28 of various length or spaces. One embodiment having a stepped arrangement of discontinuities is presented in Figure 11, with the stepped arrangement of discontinuities 28 shown in an inset figure. The embodiment of Figure 11 has multiple stepped inductive/capacitive discontinuities which are offset from each other both longitudinally and around the circumference of the antenna.

Figure 12 illustrates various combinations of monopole antenna can be employed with a matching-choke, for example to minimise size. The first embodiment shown in Figure 12 (left view) comprises a helical radiating element 30. The second embodiment shown in Figure 12 (middle view) comprises multiple slots 29 that are offset longitudinally. The third embodiment shown in Figure 12 (right view) comprises a dielectric covering 31 that covers the portion of the inner conductor that forms the radiating element. Other antenna types can be utilised to work with the same technique. Any suitable combination of antenna type and reactive element may be used.

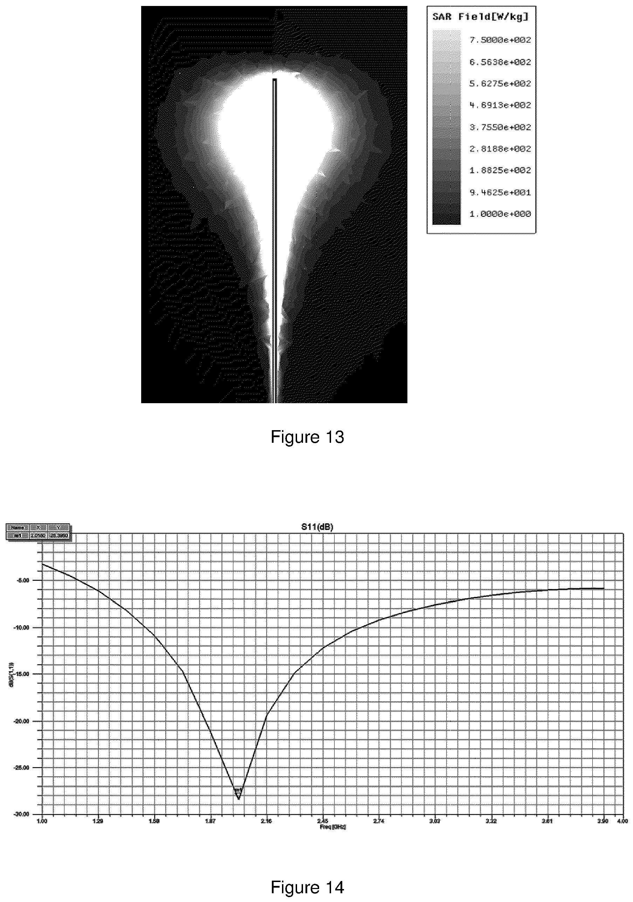

A number of slot sections can be employed to further enhance the attenuation of surface currents. An example of a typical monopole radiation pattern is presented in Figure 13. In the example of Figure 13, the monopole is a simple coaxial structure without a matching-choke element. In the computed plot of Figure 13 the specific absorption rate (SAR) represents the measure of the rate at which energy is absorbed in tissue. The plot shows SAR for a region around a monopole radiating element 100. The region 102 of highest SAR has a lachrymiform shape which extends back along the radiating element.

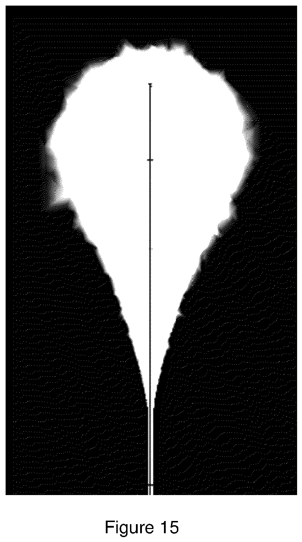

This plot has been made for a spot frequency of 2.45 GHz. In this specific example the monopole is mis-matched at the frequency of interest (2.45 GHz) as documented in the simulated S11 return loss plot in Figure 14. Line 104 represents the return loss of the antenna for which the SAR plot is shown in Figure 13. The lowest point 106 of the line is at a frequency that is lower than the desired spot frequency. The pattern of simulated necrosis for the antenna of Figures 13 and 14 is displayed in Figure 15. The region 108 of necrosis has a lachrymiform shape which extends back along the radiating element.

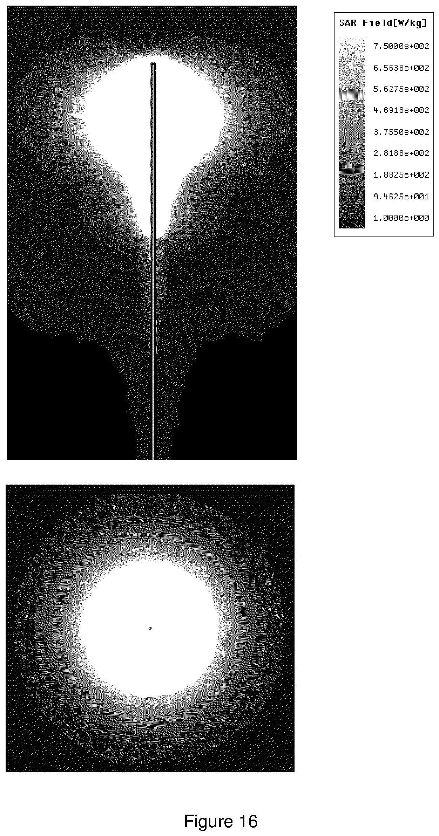

A radiation pattern for a modified version of the monopole of Figure 13 is presented in Figure 16. The monopole 110 is the same simple structure of Figure 13 but with a matching-choke element. The plot of Figure 13 plot has been made for the same spot frequency of 2.45 GHz. In this computed plot the specific absorption rate (SAR) pattern 112 is truncated in the vertical axis indicating an attenuation of surface currents on the antenna feed line surface. A second view is shown which demonstrates that the SAR pattern is substantially symmetrical around the longitudinal axis of the antenna.

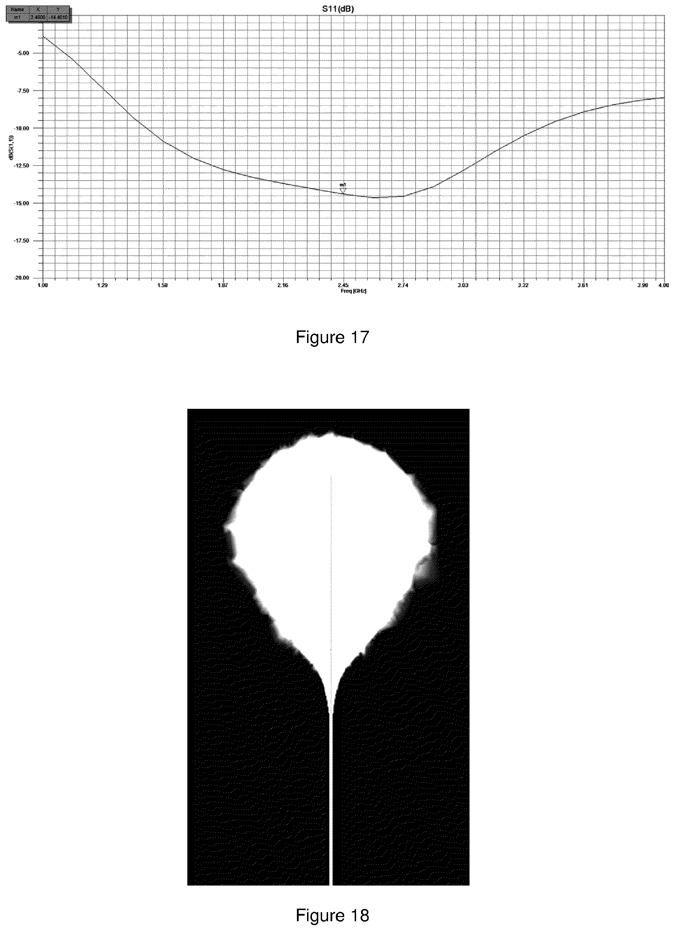

In this specific example the mis-matched monopole now has an improved match across a wide bandwidth, centred for operation at the frequency of interest (2.45 GHz) as illustrated in the simulated S11 return loss plot in Figure 17 in which line 114 represents antenna return loss. The desired frequency is shown by marker 116.

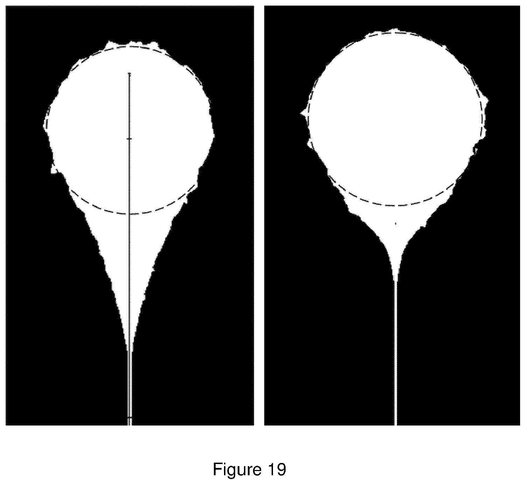

The pattern of simulated necrosis 118 for the antenna of Figures 16 and 17 is displayed in Figure 18. The pattern 118 can be seen to possess a more spherical shape when compared to the design without the matching-choke. A side-by-side comparison of the patterns of necrosis for the simple antenna 108 and for the antenna with the matching choke 118 is shown in Figure 19. An ideal spherical zone is indicated by a dashed circle 120. It may be seen that the pattern with the matching-choke is closer to the desired spherical pattern.

The shape of the necrosis region may be further optimised by the use of multiple inductive conductor/slot pairs to add additional surface current reduction. The overall design is balanced in terms of antenna mismatch, overall S11 match, and radiation pattern as all these factors interact.

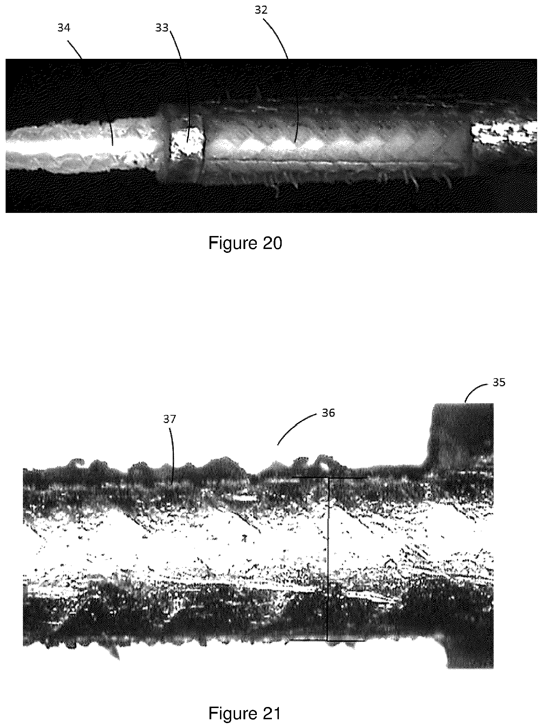

An example of a constructed monopole antenna with a matching-choke element is illustrated in Figure 20. In this example a 4 mm x 0.8 mm slot 32 has been laser removed from a 1.19 mm diameter coaxial cable (Sucoform47) leaving two 0.65 mm wide by 4mm long conductive interconnects that join to the 0.5 mm wide capacitive ring 33. The outer conductor has been fully removed to expose the inner conductor to form the radiating monopole 34. In the more detailed photo in Figure 21 the outer conductor 35 is laser ablated to expose the dielectric 36 and to leave a conductive bridge 37.

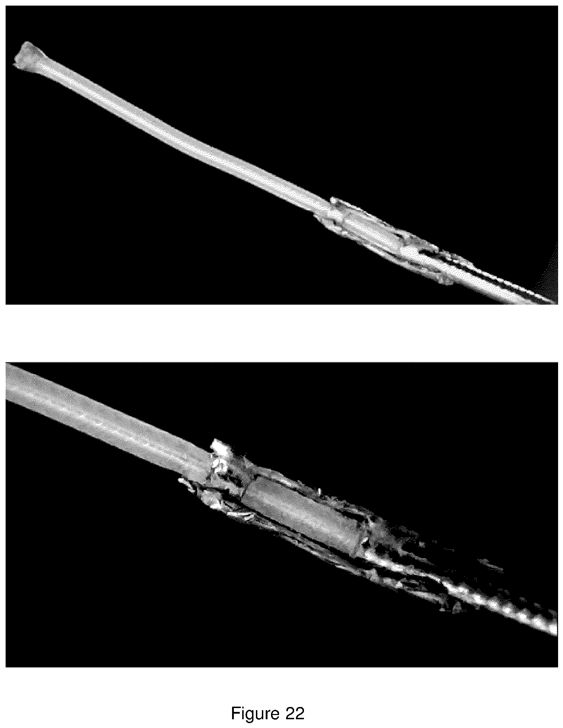

In an alternative arrangement displayed in Figure 22 a Sucoform47 cable was constructed with two 3 mm by 1 mm slots and a 1.5 mm capacitive ring to feed a 19 mm sealed monopole.

In this embodiment a different form of conductive interconnect was used instead of leaving a conductive strip of outer conductor material between slots. The conductive interconnects were two 0.45 mm (25AWG) wires attached to electrically bridge the gap creating the slots. This arrangement was explored as an alternative (cost effective) fabrication method to establish if the same performance could be achieved by other means.

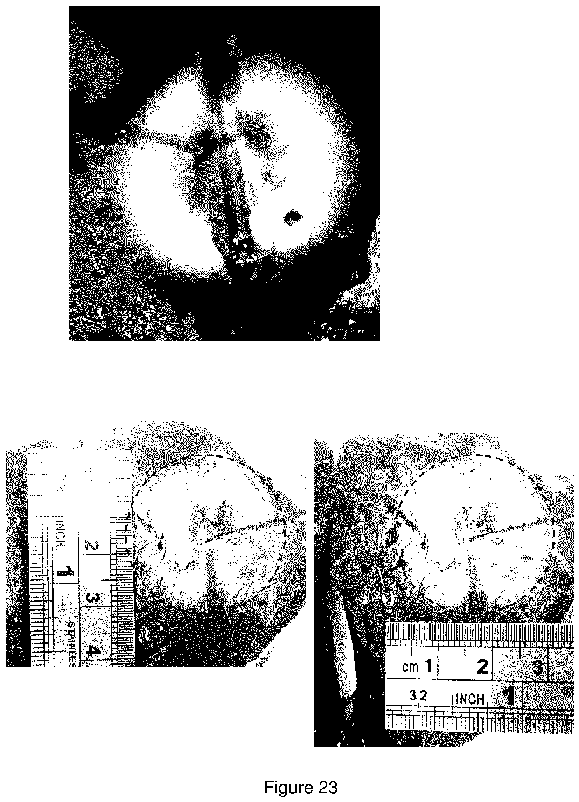

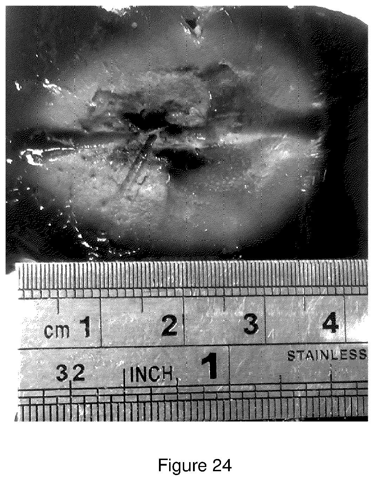

The fabricated antenna was tested in ex-vivo bovine liver (10°C storage) to determine the radiation pattern. The radiation zone in the ex-vivo tissue is displayed in Figure 23 for 70 W of 2.45 GHz microwave energy delivered. In this photograph an approximately circular 3 cm (1.18") ablation zone can be seen. The additional line from centre to outer (line at 2 o'clock in Figure 23) was made by a hole for placement of a fibre optic temperature probe (T1S-02-WNO) used to measure ablation zone temperatures. A higher power 100W ablation was run with the same antenna and displayed in Figure 24.

It has been found that methods described above may produce a more desired ablation pattern with limited surface current utilising the minimum of materials to construct a very efficient and cost effective radiator.

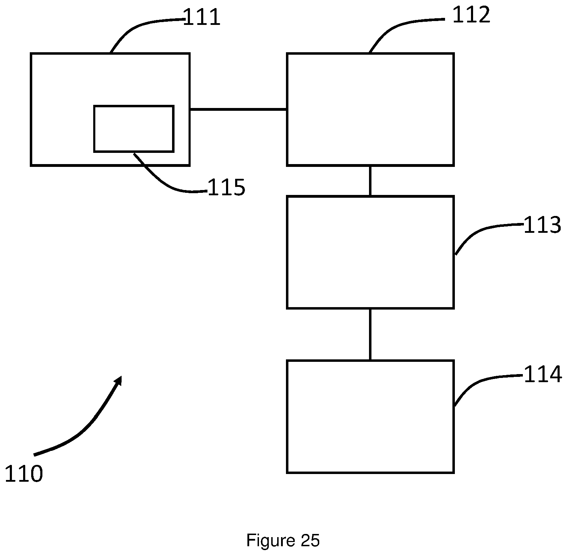

Figure 25 illustrates a microwave system generally designated 110 for treating a tissue. The microwave system 110 comprises a microwave generator 111 for providing microwave energy, a flexible interconnecting microwave cable such as a coaxial cable 112, a hand grip or hand piece 113, and a microwave antenna apparatus 114. The microwave generator 111 comprises a controller 115 configured to select a frequency of microwave energy provided to the cable apparatus and/or a power of microwave energy provided to the cable apparatus.

In embodiments, a reactive element (not shown in Figure 25) is formed on or in the microwave cable 112. The reactive element is configured to match impedances of the cable 112 and antenna apparatus 114. The reactive element may comprise any of the reactive elements described above in relation to Figures 1 to 12. The reactive element may be tuned for operation at a selected operational frequency or range of frequencies.

The microwave cable 112, the reactive element and the antenna apparatus 114 may be similar to any cable, reactive element and antenna apparatus described above with reference to any of Figures 1 to 12.

In use, the controller 115 selects an operational frequency or range of frequencies and controls the microwave generator 111 to provide microwave energy at the operational frequency or range of frequencies to the microwave cable 112.

The antenna apparatus 114 is positioned in or adjacent to tissue, for example tissue of a human patient or other subject. The antenna apparatus 114 radiates microwave energy into the tissue, causing tissue heating. The tissue heating may be such as to cause ablation.

In operation, the reactive element at least partially matches an impedance of the antenna apparatus 114 to an impedance of the microwave cable 112. The reactive element also reduces or eliminates surface currents on the microwave cable 112. Parameters of the reactive element are selected to balance matching against surface current reduction. In embodiments, a design process is used to design a reactive element, which may be referred to as a matching-choke. A radiating element (for example, a monopole antenna), a microwave feed line (for example, a coaxial cable), and a matching-choke are simulated using any suitable simulation software. Parameters of the radiating element and matching-choke are adjusted until the matching-choke substantially matches the microwave antenna to the microwave feed line while also reducing or eliminating surface currents on the microwave feed line. In some embodiments, an iterative design process is used in which the radiating element is detuned to obtain a mismatch between the radiating element and the microwave feed line at a desired frequency of operation. Parameters of the matching-choke are adjusted to compensate for the mismatch. The detuning and parameter adjustment may be repeated until values for the parameters are obtained that substantially match the microwave antenna to the microwave feed line while also reducing or eliminating surface currents on the microwave feed line. The parameters of the matching-choke may comprise, for example, slot width, slot length, slot position, number of slots, ring width, ring position, number of rings, conductive strip length, conductive strip width, conductive strip position, wire length, wire width, wire position. The parameters of the matching-choke may comprise an inductance value for at least one inductor. The parameters of the matching-choke may comprise a capacitance value for at least one capacitor.

In some circumstances, a match may change with tissue type. For example, tissue with less water, for example lung, may not be as well matched as those with higher water content, for example liver. The match may improve or degrade depending upon how the changing dielectric influences the overall design. Therefore, different designs may be used for different target dielectrics. In designing, a dielectric constant of 43 may be used for liver at 2.45 GHz. A dielectric constant of 20.5 may be used for inflated lung at 2.45 GHz. A dielectric constant of 48.4 may be used for deflated lung at 2.45 GHz.

Different designs may be used for different cable diameters.

In some circumstances, the slots may be shorted and the amount of slots may be doubled to improve attenuation. Adding further slots may have the effect of adding extra filtering elements. For example, two sets of two slots may be used.

In design, a high priority may be given to attenuating the surface waves to improve the sphericity of the pattern.

The monopole length dictates a mismatch which may be, for example, between 10 and 12 dB at the frequency of interest. In an exemplary design, increasing the slot length increases inductance. The slot conductive element 9 increases capacitance as it widens.

When the conductive element thickness is reduced the inductance increases. The overall slot length may be shortened to produce a compensatory effect of increasing the capacitance as the top end of the slot moves closer to the bottom end of the slot.

A height of the ring element 11 increases or decreases capacitance, with a taller height having greater capacitance and a shorter height having less capacitance.

In practice, the design process may start with the monopole. The ring element 11 and slot are then introduced and are optimised in length and width for a given cable diameter and tissue type. Parameters of the ring element and slot are optimised to improve match and ablation zone shape at the same time.

A target for matching may be to achieve a return loss of 12 dB to 15 dB at a frequency of interest. It has been found that designs as described above produce a very broadband frequency match. It has been found that further improvements in match may sacrifice ablation shape and vice versa.

A monopole antenna is formed from the coaxial cable. The monopole antenna comprises an exposed portion of the inner conductor of the coaxial cable. In other embodiments, an antenna may be formed from or coupled to the coaxial cable in any suitable manner.

For the coaxial cable, any suitable transmission line may be used. Any suitable reactive element may be formed in or on the transmission line.

Each

## Claims

1. A microwave apparatus comprising:
a microwave feed line configured to deliver microwave energy having a selected operational frequency or range of frequencies to a radiating element (7) extending from or coupled to a distal end of the microwave feed line;
the radiating element (7); and **characterised by**
a reactive matching-choke element formed in or on the microwave feed line;
wherein the operational frequency or range of frequencies and parameters of the reactive matching-choke element are selected such that, when the microwave apparatus is operated at the operational frequency or range of frequencies, the reactive matching-choke element both provides a desired degree of match between an impedance of the radiating element (7) and an impedance of the microwave feed line, and reduces or eliminates surface currents flowing on a ground of the feed line.

2. A microwave apparatus according to claim 1, wherein the microwave feed line comprises a coaxial cable (112), and wherein the ground comprises an outer conductor (10) of the coaxial cable (112), optionally wherein the radiating element (7) comprises an exposed distal portion of an inner conductor of the coaxial cable (112), which is longer than an outer conductor (10) of the coaxial cable (112).

3. A microwave apparatus according to claim 2, wherein the reactive matching-choke element comprises at least one aperture formed in the outer conductor (10) of the coaxial cable (112) by selective removal of part of the outer conductor (10).

4. A microwave apparatus according to claim 3, wherein the at least one aperture comprises at least one longitudinal slot (5, 5A).

5. A microwave apparatus according to claim 4, wherein at least one of a) or b):-
a) at least one conductive strip remains between the longitudinal slot or slots (5, 5A), the at least one conductive strip forming an inductive conductor element;
b) the reactive matching-choke element further comprises at least one conductive wire positioned across the at least one longitudinal slot (5, 5A), thereby forming an inductive conductor element.

6. A microwave apparatus according to claim 4 or claim 5, wherein the reactive matching-choke element comprises at least one capacitive ring (11) at a distal end of the coaxial cable (112), the or each capacitive ring (11) comprising a ring of outer conductor material remaining after formation of the at least one aperture, optionally wherein the at least one capacitive ring (11) is electrically connected to the at least one inductive conductor element.

7. A microwave apparatus according to claim 5, or claim 6 in dependence on claim 5, wherein the inductive conductor element comprises at least one discontinuity along the length of the inductive conductor element, the at least one discontinuity providing a capacitance.

8. A microwave apparatus according to any of claims 4 to 7, wherein at least one of a) to d):-
a) the at least one longitudinal slot (5, 5A) varies in width along the length of the slot (5, 5A);
b) the at least one longitudinal slot (5, 5A) has a stepped width such that different portions of the longitudinal slot (5, 5A) have different widths;
c) the at least one longitudinal slot is a single longitudinal slot (5);
d) the at least one longitudinal slot is a radially opposed pair of longitudinal slots (5, 5A).

9. A microwave apparatus according to any preceding claim, wherein the radiating element (7) comprises a monopole antenna.

10. A microwave apparatus according to any preceding claim, wherein at least one of a) and b):-
a) the surface currents comprise common mode currents;
b) the microwave apparatus is configured to perform microwave ablation of tissue and/or tissue hyperthermia at the operational frequency or range of frequencies.

11. A microwave system (110) comprising:
a microwave generator (111);
a controller (115) configured to control the microwave generator (111) to generate microwave energy having a selected operational frequency or range of frequencies;
a microwave feed line configured to deliver the microwave energy to a radiating element (7) extending from or coupled to a distal end of the microwave feed line;
the radiating element (7); and **characterised by**
a reactive matching-choke element formed in or on the microwave feed line;
wherein the operational frequency or range of frequencies and parameters of the reactive matching-choke element are selected such that, when the microwave system (110) is operated at the operational frequency or range of frequencies, the reactive matching-choke element both provides a desired degree of match between an impedance of the radiating matching-choke element and an impedance of the microwave feed line and reduces or eliminates surface currents flowing on a ground of the feed line.

12. A method comprising:
controlling a microwave generator (111) to generate microwave energy having a selected operational frequency or range of frequencies; and
delivering by a microwave feed line the microwave energy to a radiating element (7) extending from or coupled to a distal end of the microwave feed line, wherein a reactive matching-choke element is formed in or on the microwave feed line;
wherein the operational frequency or range of frequencies and parameters of the reactive matching-choke element are selected such that, when the microwave system (110) is operated at the operational frequency or range of frequencies, the reactive matching-choke element both provides a desired degree of match between an impedance of the radiating element (7) and an impedance of the microwave feed line and reduces or eliminates surface currents flowing on a ground of the feed line.

13. A method of designing a microwave apparatus, the method comprising:
simulating operation at a selected operational frequency or range of frequencies of a radiating element (7) extending from or coupled to a distal end of a microwave feed line; and
performing an iterative design procedure comprising:
simulating detuning of the radiating element (7);
selecting reactive properties of a reactive matching-choke element formed in or on the microwave feed line; and
simulating operation at the selected operational frequency or range of frequencies of the radiating element (7) in combination with the reactive matching-choke element,
the iterative design procedure being repeated for different values for parameters of the reactive matching-choke element until at the selected operational frequency or range of frequencies the reactive properties of the reactive element provide a desired degree of match between a simulated impedance of the radiating element (7) and a simulated impedance of the microwave feed line while reducing or eliminating simulated surface currents flowing on a ground of the feed line.

## Patentansprüche

1. Mikrowellenvorrichtung, Folgendes umfassend:
eine Mikrowellenzuleitung, die so konfiguriert ist, dass sie Mikrowellenenergie mit einer ausgewählten Betriebsfrequenz oder einem Frequenzbereich an ein Strahlungselement (7) liefert, das sich von einem distalen Ende der Mikrowellenzuleitung erstreckt oder mit diesem gekoppelt ist;
das Strahlungselement (7); und **gekennzeichnet durch**
ein reaktives Anpassungsdrossel-Element, das in oder an der Mikrowellenzuleitung ausgebildet ist;
wobei die Betriebsfrequenz oder der Frequenzbereich und Parameter des reaktiven Anpassungsdrossel-Elements so gewählt sind, dass, wenn die Mikrowellenvorrichtung mit der Betriebsfrequenz oder in dem Frequenzbereich betrieben wird, das reaktive Anpassungsdrossel-Element sowohl einen gewünschten Anpassungsgrad zwischen einer Impedanz des Strahlungselements (7) und einer Impedanz der Mikrowellenzuleitung bereitstellt, als auch Oberflächenströme, die auf einer Masse der Zuleitung fließen, reduziert oder beseitigt.

2. Mikrowellenvorrichtung nach Anspruch 1, wobei die Mikrowellenzuleitung ein Koaxialkabel (112) umfasst, und wobei die Masse einen Außenleiter (10) des Koaxialkabels (112) umfasst, wobei optional das Strahlungselement (7) einen freiliegenden distalen Abschnitt eines Innenleiters des Koaxialkabels (112) umfasst, der länger ist als ein Außenleiter (10) des Koaxialkabels (112).

3. Mikrowellenvorrichtung nach Anspruch 2, wobei das reaktive Anpassungsdrossel-Element mindestens eine Öffnung umfasst, die in dem Außenleiter (10) des Koaxialkabels (112) durch selektives Entfernen eines Teils des Außenleiters (10) gebildet ist.

4. Mikrowellenvorrichtung nach Anspruch 3, wobei die mindestens eine Öffnung mindestens einen Längsschlitz (5, 5A) umfasst.

5. Mikrowellenvorrichtung nach Anspruch 4, wobei mindestens eines von a) oder b) gilt:
a) mindestens ein leitender Streifen verbleibt zwischen dem Längsschlitz oder den Längsschlitzen (5, 5A), wobei der mindestens eine leitende Streifen ein induktives Leiterelement bildet;
b) das reaktive Anpassungsdrossel-Element umfasst ferner mindestens einen leitenden Draht, der über dem mindestens einen Längsschlitz (5, 5A) positioniert ist, wodurch ein induktives Leiterelement gebildet wird.

6. Mikrowellenvorrichtung nach Anspruch 4 oder Anspruch 5, wobei das reaktive Anpassungsdrossel-Element mindestens einen kapazitiven Ring (11) an einem distalen Ende des Koaxialkabels (112) umfasst, wobei der oder jeder kapazitive Ring (11) einen Ring aus Außenleitermaterial umfasst, der nach der Bildung der mindestens einen Öffnung verbleibt, wobei der mindestens eine kapazitive Ring (11) optional mit dem mindestens einen induktiven Leiterelement elektrisch verbunden ist.

7. Mikrowellenvorrichtung nach Anspruch 5 oder Anspruch 6 in Abhängigkeit von Anspruch 5, wobei das induktive Leiterelement mindestens eine Diskontinuität entlang der Länge des induktiven Leiterelements umfasst, wobei die mindestens eine Diskontinuität eine Kapazität bereitstellt.

8. Mikrowellenvorrichtung nach einem der Ansprüche 4 bis 7, wobei mindestens eines von a) bis d) gilt:
a) die Breite des mindestens einen Längsschlitzes (5, 5A) variiert entlang der Länge des Schlitzes (5, 5A);
b) der mindestens eine Längsschlitz (5, 5A) weist eine gestufte Breite auf, sodass verschiedene Abschnitte des Längsschlitzes (5, 5A) unterschiedliche Breiten aufweisen;
c) der mindestens eine Längsschlitz ist ein einzelner Längsschlitz (5);
d) der mindestens eine Längsschlitz besteht aus einem radial gegenüberliegenden Paar von Längsschlitzen (5, 5A).

9. Mikrowellenvorrichtung nach einem der vorhergehenden Ansprüche, wobei das Strahlungselement (7) eine Monopolantenne umfasst.

10. Mikrowellenvorrichtung nach einem der vorhergehenden Ansprüche, wobei mindestens eines von a) und b) gilt:
a) die Oberflächenströme umfassen Gleichtaktströme;
b) die Mikrowellenvorrichtung ist so konfiguriert, dass sie eine Mikrowellenablation von Gewebe und/oder eine Gewebehyperthermie mit der Betriebsfrequenz oder in dem Frequenzbereich durchführt.

11. Mikrowellensystem (110), Folgendes umfassend:
einen Mikrowellengenerator (111);
eine Steuerung (115), die so konfiguriert ist, dass sie den Mikrowellengenerator (111) so steuert, dass er Mikrowellenenergie mit einer ausgewählten Betriebsfrequenz oder einem ausgewählten Frequenzbereich erzeugt;
eine Mikrowellenzuleitung, die so konfiguriert ist, dass sie die Mikrowellenenergie an ein Strahlungselement (7) liefert, das sich von einem distalen Ende der Mikrowellenzuleitung aus erstreckt oder mit diesem gekoppelt ist;
das Strahlungselement (7); und **gekennzeichnet durch**
ein reaktives Anpassungsdrossel-Element, das in oder an der Mikrowellenzuleitung ausgebildet ist;
wobei die Betriebsfrequenz oder der Frequenzbereich und Parameter des reaktiven Anpassungsdrossel-Elements so gewählt sind, dass, wenn das Mikrowellensystem (110) mit der Betriebsfrequenz oder in dem Frequenzbereich betrieben wird, das reaktive Anpassungs-Drosselelement sowohl einen gewünschten Anpassungsgrad zwischen einer Impedanz des strahlenden Anpassungsdrossel-Elements und einer Impedanz der Mikrowellenzuleitung bereitstellt, als auch Oberflächenströme, die auf einer Masse der Zuleitung fließen, reduziert oder beseitigt.

12. Verfahren, Folgendes umfassend:
Steuern eines Mikrowellengenerators (111) zum Erzeugen von Mikrowellenenergie mit einer ausgewählten Betriebsfrequenz oder einem ausgewählten Frequenzbereich; und
Liefern der Mikrowellenenergie, durch eine Mikrowellenzuleitung, an ein Strahlungselement (7), das sich von einem distalen Ende der Mikrowellenzuleitung aus erstreckt oder mit diesem gekoppelt ist, wobei ein reaktives Anpassungsdrossel-Element in oder auf der Mikrowellenzuleitung ausgebildet ist;
wobei die Betriebsfrequenz oder der Frequenzbereich und Parameter des reaktiven Anpassungsdrossel-Elements so gewählt sind, dass, wenn das Mikrowellensystem (110) mit der Betriebsfrequenz oder in dem Frequenzbereich betrieben wird, das reaktive Anpassungsdrossel-Element sowohl einen gewünschten Anpassungsgrad zwischen einer Impedanz des Strahlungselements (7) und einer Impedanz der Mikrowellenzuleitung bereitstellt, als auch Oberflächenströme, die auf einer Masse der Zuleitung fließen, reduziert oder beseitigt.

13. Verfahren zum Entwerfen einer Mikrowellenvorrichtung, wobei das Verfahren Folgendes umfasst:
Simulieren des Betriebs mit einer ausgewählten Betriebsfrequenz oder in einem ausgewählten Frequenzbereich eines Strahlungselements (7), das sich von einem distalen Ende einer Mikrowellenzuleitung aus erstreckt oder mit diesem gekoppelt ist; und
Durchführen einer iterativen Entwurfsprozedur, Folgendes umfassend:
Simulieren einer Verstimmung des Strahlungselements (7);
Auswählen von reaktiven Eigenschaften eines reaktiven Anpassungsdrossel-Elements, das in oder an der Mikrowellenzuleitung ausgebildet ist; und
Simulieren des Betriebs mit der ausgewählten Betriebsfrequenz oder in dem ausgewählten Frequenzbereich des Strahlungselements (7) in Kombination mit dem reaktiven Anpassungsdrossel-Element,
wobei die iterative Entwurfsprozedur für verschiedene Werte für Parameter des reaktiven Anpassungsdrossel-Elements wiederholt wird, bis bei der ausgewählten Betriebsfrequenz oder in dem ausgewählten Frequenzbereich die reaktiven Eigenschaften des reaktiven Elements einen gewünschten Anpassungsgrad zwischen einer simulierten Impedanz des Strahlungselements (7) und einer simulierten Impedanz der Mikrowellenzuleitung bereitstellen, während simulierte Oberflächenströme, die auf einer Masse der Zuleitung fließen, reduziert oder beseitigt werden.

## Revendications

1. Appareil à micro-ondes comprenant :
une ligne d'alimentation de micro-ondes configurée pour délivrer de l'énergie de micro-ondes ayant une fréquence ou une plage de fréquences de service sélectionnée à un élément radiant (7) s'étendant depuis ou étant couplé à une extrémité distale de la ligne d'alimentation de micro-ondes ;
l'élément radiant (7) ; et **caractérisé par**
un élément réactif d'adaptation et de bobine d'arrêt formé dans ou sur la ligne d'alimentation de micro-ondes ;
dans lequel la fréquence ou la plage de fréquences de service et des paramètres de l'élément d réactif d'adaptation et de bobine d'arrêt sont sélectionnés de sorte que, lorsque l'appareil à micro-ondes fonctionne à la fréquence ou la plage de fréquences de service, l'élément réactif d'adaptation et de bobine d'arrêt à la fois fournit un degré souhaité d'adaptation entre une impédance de l'élément radiant (7) et une impédance de la ligne d'alimentation de micro-ondes, et réduit ou élimine des courants de surface circulant sur une masse de la ligne d'alimentation.

2. Appareil à micro-ondes selon la revendication 1, dans lequel la ligne d'alimentation de micro-ondes comprend un câble coaxial (112), et dans lequel la masse comprend un conducteur extérieur (10) du câble coaxial (112), optionnellement dans lequel l'élément radiant (7) comprend une partie distale exposée d'un conducteur intérieur du câble coaxial (112), qui est plus long qu'un conducteur extérieur (10) du câble coaxial (112).

3. Appareil à micro-ondes selon la revendication 2, dans lequel l'élément réactif d'adaptation et de bobine d'arrêt comprend au moins une ouverture formée dans le conducteur extérieur (10) du câble coaxial (112) par un enlèvement sélectif d'une partie du conducteur extérieur (10).

4. Appareil à micro-ondes selon la revendication 3, dans lequel l'au moins une ouverture comprend au moins une fente longitudinale (5, 5A).

5. Appareil à micro-ondes selon la revendication 4, dans lequel dans lequel au moins un parmi a) ou b) s'applique :
a) au moins un ruban conducteur demeure entre la/les fente(s) longitudinale(s) (5, 5A), l'au moins un ruban conducteur formant un élément conducteur inducteur ;
b) l'élément réactif d'adaptation et de bobine d'arrêt comprend en outre au moins un fil conducteur positionné sur l'au moins une fente longitudinale (5, 5A), en formant ainsi un élément conducteur inducteur.

6. Appareil à micro-ondes selon la revendication 4 ou la revendication 5, dans lequel l'élément réactif d'adaptation et de bobine d'arrêt comprend au moins une bague capacitive (11) à une extrémité distale du câble coaxial (112), la ou chaque bague capacitive (11) comprenant une bague d'un matériau conducteur extérieur demeurant après la formation de l'au moins une ouverture, optionnellement dans lequel l'au moins une bague capacitive (11) est connectée électriquement à l'au moins un élément conducteur inducteur.

7. Appareil à micro-ondes selon la revendication 5 ou la revendication 6 dépendant de la revendication 5, dans lequel l'élément conducteur inducteur comprend au moins une discontinuité le long de la longueur de l'élément conducteur inducteur, l'au moins une discontinuité fournissant une capacitance.

8. Appareil à micro-ondes selon l'une quelconque des revendications 4 à 7, dans lequel au moins un parmi a) à d) s'applique :
a) l'au moins une fente longitudinale (5, 5A) varie en largeur le long de la longueur de la fente (5, 5A) ;
b) l'au moins une fente longitudinale (5, 5A) a une largeur échelonnée de telle sorte que différentes parties de la fente longitudinale (5, 5A) ont différentes largeurs ;
c) l'au moins une fente longitudinale est une fente longitudinale unique (5) ;
d) l'au moins une fente longitudinale est une paire radialement opposée de fentes longitudinales (5, 5A).

9. Appareil à micro-ondes selon l'une quelconque des revendications précédentes, dans lequel l'élément radiant (7) comprend une antenne unipolaire.

10. Appareil à micro-ondes selon l'une quelconque des revendications précédentes, dans lequel au moins un parmi a) et b) s'applique :
a) les courants de surface comprennent des courants de mode commun ;
b) l'appareil à micro-ondes est configuré pour réaliser une ablation par micro-ondes de tissu et/ou une hyperthermie de tissu à la fréquence ou la plage de fréquences de service.

11. Système de micro-ondes (110) comprenant :
un générateur de micro-ondes (111) ;
un dispositif de commande (115) configuré pour commander le générateur de micro-ondes (111) pour générer de l'énergie de micro-ondes ayant une fréquence ou une plage de fréquences de service sélectionnée ;
une ligne d'alimentation de micro-ondes configurée pour délivrer l'énergie de micro-ondes à un élément radiant (7) s'étendant depuis ou étant couplé à une extrémité distale de la ligne d'alimentation de micro-ondes ;
l'élément radiant (7) ; et **caractérisé par** un
un élément réactif d'adaptation et de bobine d'arrêt formé dans ou sur la ligne d'alimentation de micro-ondes ;
dans lequel la fréquence ou la plage de fréquences de service et des paramètres de l'élément réactif d'adaptation et de bobine d'arrêt sont sélectionnés de sorte que, lorsque le système de micro-ondes (110) fonctionne à la fréquence ou la plage de fréquences de service, l'élément réactif d' adaptation et de bobine d'arrêt à la fois fournit un degré souhaité d'adaptation entre une impédance de l'élément réactif d'adaptation et de bobine d'arrêt et une impédance de la ligne d'alimentation de micro-ondes, et réduit ou élimine des courants de surface circulant sur une masse de la ligne d'alimentation.

12. Procédé comprenant :
la commande d'un générateur de micro-ondes (111) pour générer de l'énergie de micro-ondes ayant une fréquence ou une plage de fréquences de service sélectionnée ; et
la délivrance, par une ligne d'alimentation de micro-ondes, de l'énergie de micro-ondes à un élément radiant (7) s'étendant depuis ou étant couplé à une extrémité distale de la ligne d'alimentation de micro-ondes, dans lequel un élément réactif d'adaptation et de bobine d'arrêt est formé dans ou sur la ligne d'alimentation de micro-ondes ;
dans lequel la fréquence ou la plage de fréquences de service et des paramètres de l'élément réactif d'adaptation et de bobine d'arrêt sont sélectionnés de sorte que, lorsque le système de micro-ondes (110) fonctionne à la fréquence ou la plage de fréquences de service, l'élément réactif d' adaptation et de bobine d'arrêt à la fois fournit un degré souhaité d'adaptation entre une impédance de l'élément radiant (7) et une impédance de la ligne d'alimentation de micro-ondes, et réduit ou élimine des courants de surface circulant sur une masse de la ligne d'alimentation.

13. Procédé de conception d'un appareil de micro-ondes, le procédé comprenant :
la simulation du fonctionnement à une fréquence ou une plage de fréquences de service sélectionnée d'un élément radiant (7) s'étendant depuis ou étant couplé à une extrémité distale d'une ligne d'alimentation de micro-ondes ; et
la réalisation d'une procédure de conception itérative comprenant :
la simulation d'un désaccordement de l'élément radiant (7) ;
la sélection de propriétés réactives d'un élément réactif d'adaptation et de bobine d'arrêt formé dans ou sur la ligne d'alimentation de micro-ondes ; et
la simulation du fonctionnement à la fréquence ou la plage de fréquences de service sélectionnée de l'élément radiant (7) en combinaison avec l'élément réactif d'adaptation et de bobine d'arrêt,
la procédure de conception itérative étant répétée pour différentes valeurs de paramètres de l'élément réactif d'adaptation et de bobine d'arrêt jusqu'à ce qu'à la fréquence ou la plage de fréquences de service sélectionnée, les propriétés réactives de l'élément réactif d'adaptation et de bobine d'arrêt fournissent un degré souhaité d'adaptation entre une impédance simulée de l'élément radiant (7) et une impédance simulée de la ligne d'alimentation de micro-ondes tout en réduisant ou éliminant des courants de surface simulés circulant sur une masse de la ligne d'alimentation.
